Europäisches Patentamt

European Patent Office

Office européen des brevets

(19)

(11) Publication number: 0 316 659
A2

# (12) EUROPEAN PATENT APPLICATION

(21) Application number: 88118271.1

(22) Date of filing: 02.11.88

(51) Int. Cl.4: B41F 17/00

(30) Priority: 13.11.87 US 120420

(43) Date of publication of application:
24.05.89 Bulletin 89/21

(84) Designated Contracting States:
AT BE CH DE ES FR GB GR IT LI LU NL SE

(71) Applicant: Levin, Hanan
1871 Golden Gate Avenue
San Fransisco California 94115(US)

Applicant: Huntington, Maura
1871 Golden Gate Avenue
San Fransisco California 94115(US)

(72) Inventor: Levin, Hanan
1871 Golden Gate Avenue
San Fransisco California 94115(US)
Inventor: Huntington, Maura
1871 Golden Gate Avenue
San Fransisco California 94115(US)

(74) Representative: Casalonga, Axel et al
BUREAU D.A. CASALONGA - JOSSE
Morassistrasse 8
D-8000 Munich 5(DE)

(54) Printed condom manufacturing system.

(57) A printed condom manufacturing system pro-
duces a condom with printing which is sealed within
the material forming the condom. The condom print-
ing system has a first material application station, a
printing station and a second material application
station. A first layer of condom forming material is
applied to a form in the first material application
station and then printing is applied to the first layer
of material in the printing station. After printing is
completed a second substantially transparent layer
of condom forming material is applied in the second
material application station covering the first layer of
material and the printing thereon.

FIG. 2.

## PRINTED CONDOM MANUFACTURING SYSTEM

### Background of the Invention

The invention relates to systems for manufacturing prophylactic condoms, and more particularly to an improved method and apparatus for producing condoms which are provided with indicia such as printing or designs.

Condoms are commonly made from latex rubber in a highly automated process. Commonly, a continuous conveyor system carries a number of generally phallic-shaped molds on which the condoms are formed. The molds are first dipped in a tank containing liquid latex rubber with additives to achieve desirable qualities. The molds are then withdrawn from the tank covered with a thin layer of the latex material and are passed through drying or curing ovens. After curing the molds may be dipped a second time in a tank containing liquid latex to deposit a second layer of latex on the mold. The molds then pass through a device which rolls a ring on the open end of the condom and then through another drying or curing oven. The condom may then be processed further by applying lubricants or spermicides prior to or concurrently with packaging.

Although different coloring agents may be added to the liquid latex to produce condoms of different colors, condoms are generally monochromatic. This is due to the above described method of manufacture. Since the latex is deposited on the molds by dipping in liquid latex, the color of the finished product is generally of uniform color corresponding to the color of the liquid latex.

There have been devices which print on a condom generally during the manufacturing process. U.S. Patent Nos. 2,244,592, 2,221,711, and 2,221,323 all disclose devices for applying some indicia to a condom or other rubber good formed on a mold. However, these devices apply printing or other indicia only to the outside of the finished or substantially finished product. The print material is therefore exposed on the outside surface of the condom where it may be erased or damaged by lubricants or spermicides applied prior to packaging.

Condoms are invasive birth-control and protective devices and therefore must meet rigorous FDA regulations designed to prevent health hazards which may arise from their use. Since prior systems leave the printing medium exposed on the outer surface of the condom, there is the possibility of health hazards arising from exposure to the print medium.

### Summary of the Invention

Pursuant to the present invention a condom is provided with printing which is not exposed on its outer surface, thereby preventing the possibility of health hazards from exposure to the print medium. The condom has a first layer of condom material upon which printing is applied. A second layer of condom material covers the print medium and the first layer of condom material so that the print medium is entirely sealed between the two layers of material.

The condom is formed on a generally phallic-shaped form or mandrel. A first layer of condom material is applied to the mandrel and printing is applied to the first layer of material. After printing is applied, a second layer of condom material is applied over the first layer of material and the printing. The printing is therefore sealed between the first and second layers of material which form the condom.

The first layer of material may be latex rubber and may be applied by dipping the mandrel in a tank of liquid latex rubber with appropriate additives. Upon removal from the tank a thin layer of latex covers the mandrel. The first layer of latex must then be cured until it is stable enough to support the printing application. Curing may be done in a curing oven of infra-red or other design.

Printing for purposes of this invention may include letters, words, designs, or any other type of indicia or image. The printing may be applied in any suitable way such as rotary press printing, roto-gravure, silk-screen printing, pad-printing, or ink-jet printing, either individually or in some combination. The printing may be applied for example with a rotary press using a flexographic drum to transfer the particular indicia to the first layer of material. In this case different colors would be transferred by different flexographic drums to produce the intended image.

The printing medium can be any suitable for the particular image transfer system. Curing may also be required for the printing medium after application, by ultra-violet or other appropriate curing means.

The second layer of material can be applied by again dipping the mandrel, having the first layer of latex with the desired printing on its outer surface, into a tank of liquid latex. The second layer of latex is substantially transparent so that the printing is visible through the second layer. After the second layer is applied and the mandrel removed from the tank, a ring may be formed on the open end of the condom as is conventional in condom manufacturing and then both layers must be fully cured.

A continuous conveyor system preferably carries a number of mandrels through the steps described above. The conveyor system must include means for manipulating the mandrels such as dipping them into the latex, holding them in position through the curing ovens and through the printing system, or allowing the mandrels to rotate as required through the various manufacturing steps.

A broad object of the present invention is to provide a condom having printing which is completely encapsulated in the condom forming material so that the print medium is not exposed to the user or the user's partner.

Another object of the present invention is to provide a method for producing a condom having sealed printing.

Yet another object of the present invention is to provide apparatus for producing a condom having printing which is sealed within the condom material.

These and other objects, advantages and features of the invention will be apparent from the following description of preferred embodiments, considered along with the accompanying drawings.

Brief Description of the Drawings

Figure 1 is a flow chart showing the general arrangement and sequence of the present printing system.

Figure 2 is a partially diagrammatic representation of a preferred embodiment of the present condom printing system.

Description of the Preferred Embodiments

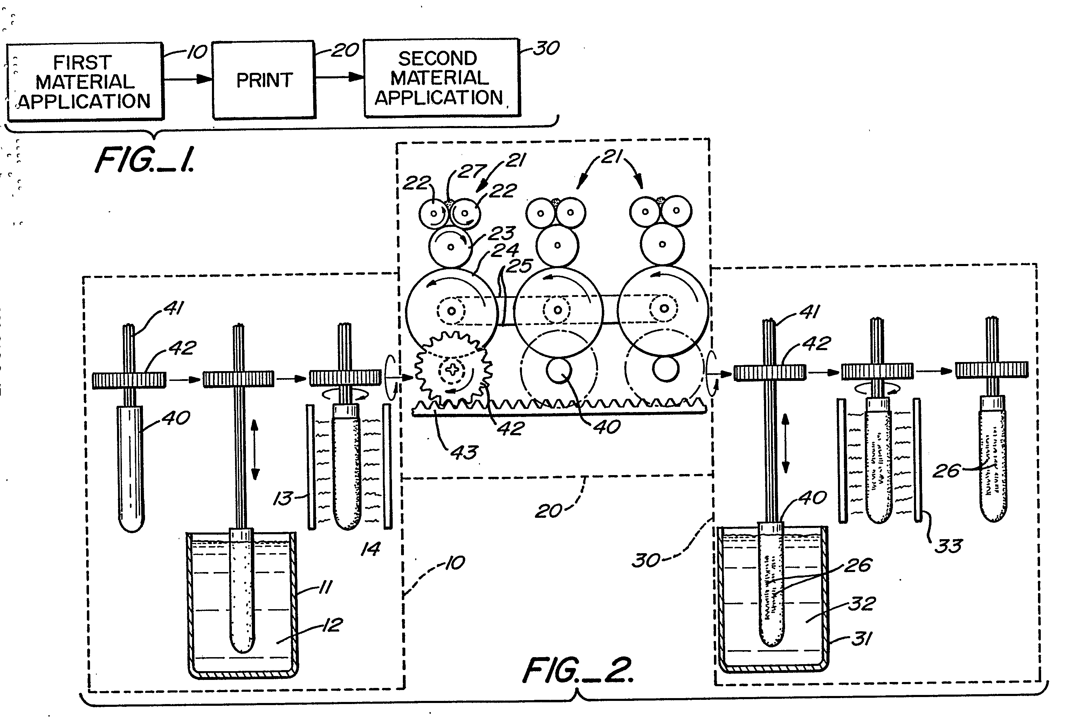

Referring to Figure 1, the manufacturing system pursuant to the present invention includes a first material application station 10, a printing station 20, and a second material application station 30. A first layer of material is formed in the first material application station. The first layer of material is then transported to the printing station where the desired printing is applied. The printed first layer is then transported to the second material application station where a second layer of material is applied covering the first layer and the printing. The second layer of material is substantially transparent so that the printing is visible therethrough but not exposed.

In a preferred embodiment shown in Figure 2, the first material application station 10 includes a tank 11 containing liquid latex 12 and a curing oven 13.

The printing station 20 includes a rotary press having several printers 21 for printing different colors on the condom which make up the finished image or indicia. Each printer includes nip rollers 22, ink transfer roller 23, and flexographic printing drum 24. A print medium or ink 27 is metered from a reservoir (not shown) to the nip rollers. A timing link 25 between the printers keeps the flexographic drums timed so that the image transferred by each aligns properly on the condom with the images transferred by the other drums.

The second material application station 30 includes a second tank 31 containing liquid latex 32 and a final curing oven 33.

The system also includes generally phallic-shaped forms or mandrels 40 which are each carried on splined shaft 41. The mandrels in this embodiment may have an outer surface of flexible silicon material to facilitate printing on the first layer of latex material. Each splined shaft extends through a spur gear 42 so that as the spur gear turns it rotates the splined shaft and mandrel about their longitudinal axes. Preferably the splined shafts and mandrels are transported through the system on a continuous conveyor system (not shown) which manipulates the mandrels and carries them through each station of the system.

The operation of one embodiment of the present invention may now be described with reference to the Figures. A mandrel 40 is first passed through the first material application station 10. The mandrel is immersed to a point in the liquid latex 12 contained in the latex tank 11. A circumferential groove (not shown) may be cut into the mandrel at a point along its length corresponding to the depth of immersion, to form a thickened ring at the open end of the condom. After the mandrel is removed from the liquid latex a thin layer of latex 14 remains on the mandrel which is then cured to some extent as the mandrel passes through the first curing oven 13 which may be an infra-red oven.

After the first layer of latex on the mandrel is sufficiently cured to withstand printing pressure, the mandrel is transported to the printing station 20. As the mandrel is transported past the first printer 21, an inked surface of the flexographic printing drum contacts the first layer of latex on the mandrel and transfers an image to the surface of the latex.

In the illustrated embodiment, nip rollers 22 roll latex ink 27 onto an ink transfer roller 23 which in turn places a proper amount of the ink on the flexographic printing drum 24. The flexographic drum has a printing plate (not shown) having a raised pattern which takes the ink from the transfer roller and which forms the image to be transferred.

After the first printing the mandrel passes on to the next printers where other images each having a different color are transferred to the first layer of latex. The images transferred to the latex make up the desired image having in this embodiment as many as four different colors, the base latex color and three other colors applied by the three printers 21.

As the mandrels pass through the printing station the spur gear contacts and rolls along the spur gear rack 43 to maintain the position of the mandrels to be contacted by the printing drums 24. The angular velocity of the mandrels and the printing drums are coordinated such that the image from the printing drum is transferred cleanly to the latex without slipping and blurring. The rotation of the printing drums is coordinated by timing link 25 so that each image transferred is in proper alignment with the other images.

In this embodiment it can be seen that the printing can cover the entire surface of the first latex layer, over its entire circumference and over its length. Furthermore, the image may include many different colors with the addition of more printers.

After the desired image 26 has been transferred to the first layer of latex 14 covering the mandrel 40, the mandrel is then transported to the second material application station 30. The mandrel is immersed in liquid latex 32 contained in latex tank 31 and then removed having a second layer of latex covering the first layer and the printed image 26. The second layer of latex is substantially transparent so that the image is readily visible. Both layers of latex are then completely cured in curing oven 33 prior to removal from the mandrel and packaging or further processing before packaging. The curing oven may be an infra-red or other suitable type of curing oven.

The system may also include means for forming a ring of material at the open end of the layers of material prior to final curing. In a preferred embodiment the means may be located in the second material application station after the second latex tank.

In the illustrated embodiment the transport system manipulates the mandrels to immerse them in the latex tanks and hold them in the curing ovens as well as in the printing station. In a preferred embodiment the mandrels are immersed by extending the splined shafts as the mandrels pass over the latex tanks. Other embodiments might have the angular orientation of the shaft changed to dip the mandrels in the tanks. Also in the preferred embodiment the transport system rotates the mandrels about their longitudinal axes while passing them through the latex tanks and also through the curing ovens. The mandrels are also rotated about an axis perpendicular to the longitudinal axis of the mandrels so that the mandrels are horizontal while passing through the printing station and then rotated again to be substantially vertical while passing through the latex tanks and curing ovens.

In other embodiments the mandrels may be manipulated differently through the various stations. For example the mandrels may not be rotated through the latex tanks or may pass through the printing station vertically. Also, while a rotary press printer is illustrated in Figure 2, many other types of image transferring devices may also be employed such as ink jet printers, litho or plate printers, pad printers, silk-screen printers, and roto-gravure printers as well as combinations of these.

In litho or plate printing the mandrel rolls across an inked plate that has been etched to correspond to the desired image to be transferred. A series of notches markers at the base of the mandrel secure color-to-color accuracy and registration. For printing more than one color a series of plates may by used each with a different color or one longer plate which is segmented into different colors, upon which the mandrel rolls.

Screen printing utilizes a screen having openings corresponding to the image to be transferred is brought into contact with the first layer on the mandrel. A squeegee then moves across the screen depositing ink through the openings. Several such screen prints of different colors may be necessary to form the desired image with a curing pause between each application.

In pad printing a soft pad is brought into contact with an etched cliche plate which has been inked. The contact with the plate transfers an image to the pad and the pad is then brought into contact with the first layer of material on the mandrel leaving an imprint. A plurality of pad printers are used for multi-color printing.

Ink-jet printing sprays ink in a pattern that was numerically plotted on a computer and translated to the jets. Thus in this method only the ink comes in physical contact with the first layer of material on the mandrel.

In roto-gravure printing a cylinder has the desired image etched on its surface in the form of cells and wells and an impression cylinder. The impression cylinder contacts the cliche plate and picks up ink forming the image to be transferred. The impression cylinder then rolls against the first layer of material leaving the desired image.

The type of ink or print medium is not limited to latex ink. Other mediums suitable to the particular printing mechanism or mechanisms may be employed. However some print mediums may require some means for curing the medium prior to the second material application or between color applications. The print medium may be cured by chemicals, ultra-violet light or other appropriate method for the particular medium which can be tolerated by the latex. For example silk-screening printers would require curing between print medium applications.

The above described preferred embodiments are intended to illustrate the principles of the invention, but not to limit the scope of the invention. Various other embodiments and modifications to these preferred embodiments may be made by those skilled in the art, without departing from the scope of the following claims.

## Claims

1. A method for printing on a condom wherein the condom is formed on a generally phallic-shaped form, comprising the steps:
applying a first layer of condom material on the phallic-shaped form;
applying printing to at least a portion of the outer surface of the first layer of condom material;
applying a second layer of condom material over the first layer having the printing thereon, the second layer of condom material being substantially transparent such that the printing on the first layer is visible through the second layer.

2. The method of claim 1 wherein the first and second layers of condom material are latex rubber and the first layer is applied by dipping the form into a first tank of liquid latex rubber and the second layer is formed by dipping the form into a second tank of liquid latex rubber.

3. The method of claim 2 including the step of rotating the form about its longitudinal axis while it is in the liquid latex rubber.

4. The method of claim 2 including the step of curing the first layer of latex sufficiently to withstand the application of printing on its surface by passing the form having the first layer thereon through a first curing oven.

5. The method of claim 2 including the step of thoroughly curing the first and second layers of latex by passing the form including the first layer with the printing thereon and the second layer through a second curing oven.

6. The method of claim 5 including the step of rotating the form about its longitudinal axis as it passes through the first and second curing ovens.

7. The method of claim 1 wherein the printing covers substantially the entire surface of the first layer of condom material.

8. The method of claim 1 wherein the printing is applied by a flexographic rotary press.

9. The method of claim 1 wherein the printing includes a plurality of colors.

10. The method of claim 1 including the step of curing the print medium prior to applying the second layer of condom material.

11. A printed condom manufactured by the method of claim 1.

12. A condom, which comprises:
a first layer of condom material;
printing applied on at least a portion of the first layer of condom material;
a second layer of substantially transparent condom material covering the first layer and the printing thereon so that the printing is visible through the second layer of material but none of the printing is exposed on the outside surface of the condom.

13. The condom as defined in claim 12 wherein the condom material is latex rubber.

14. An apparatus for manufacturing a condom having printing thereon wherein the condom is formed on a generally phallic-shaped form, comprising:
first condom material application means for applying a first layer of condom material on the form;
printing means for applying a printing substance over at least a portion of the outer surface of the first layer of condom material; and
second condom material application means for applying a second substantially transparent layer of condom material on the form over the first layer having the printing thereon, wherein the printing material on the first layer of condom material is covered by the second layer of condom material.

15. The apparatus of claim 14 wherein the condom material is latex rubber.

16. The apparatus of claim 15, wherein the first material application means includes a first tank containing liquid latex rubber and a first curing means for curing the first layer of latex sufficiently to support printing thereon, the second material application means includes a second tank containing liquid latex and a second curing means for thoroughly curing the first and second layers of latex, and wherein the layers of latex are applied to the phallic-shaped form by dipping it into the liquid latex.

17. The apparatus of claim 16, including means for rotating the forms about their longitudinal axes as they pass through the first and second tanks containing latex and as they pass through the first and second curing means.

18. The apparatus of claim 16, wherein the first and second curing means are infrared ovens.

19. The apparatus of claim 14, wherein the printing means includes a rotary press having at least one flexographic drum for transferring an image to the outer surface of the first layer of condom material.

20. The apparatus of claim 14, wherein the printing means includes curing means for curing the print medium.

21. The apparatus of claim 14, including conveyor means for conveying a plurality of the generally phallic-shaped forms through the first material application means, the printing means, and the second material application means so that layers of condom material and the printing can be applied.

EP 0 316 659 A2

FIRST MATERIAL APPLICATION —10→ PRINT —20→ SECOND MATERIAL APPLICATION —30

FIG._1.

FIG._2.